# EUROPÄISCHE PATENTANMELDUNG

(11) **EP 4 763 042 A1**
(43) Veröffentlichungstag der Anmeldung: **24.06.2026**
(21) Anmeldenummer: 25222854.9
(22) Anmeldetag: 12.12.2025
(51) Int. Cl.: A61B 1/00, A61B 1/05

(54) **BILDGEBUNGSVORRICHTUNG, BILDGEBUNGSSYSTEM UND VERFAHREN ZU EINEM BETRIEB EINER BILDGEBUNGSVORRICHTUNG**

(30) Priorität: 20.12.2024 DE 102024139198
(71) Anmelder: Karl Storz SE & Co. KG, 78532 Tuttlingen (DE)
(72) Erfinder: BARTH, Sebastian, 78532 Tuttlingen (DE); Bauer, Franz, 78532 Tuttlingen (DE)

(57) **Zusammenfassung**

Die Erfindung betrifft eine Bildgebungsvorrichtung (10; 110; 210), insbesondere eine Endoskopvorrichtung, mit einem Schaft (12; 112; 212), welcher eine Längsachse (14; 114; 214) definiert, und mit einer Stereoskopieeinheit (16; 116; 216) zur stereoskopischen Bilderzeugung.

Es wird vorgeschlagen, dass die Stereoskopieeinheit (16; 116; 216) eine Bewegungseinheit (20; 120; 220) zu einer mechanisch bewirkten Änderung eines Auswahlbereichs (22, 22'; 122, 122'; 222, 222') aus einem Sichtfeld (24; 124; 224) der Stereoskopieeinheit (16; 116; 216) für eine Bilderfassung aufweist, wobei die Stereoskopieeinheit (16; 116; 216) dazu eingerichtet ist, den Auswahlbereich (22, 22'; 122, 122'; 222, 222') zur Erfassung von Bildern zur Nutzung bei der stereoskopischen Bilderzeugung in Abhängigkeit von einem Rotationsparameter bezüglich der Längsachse (14; 114; 214) festzulegen.

## Beschreibung

Die vorliegende Erfindung betrifft eine Bildgebungsvorrichtung zur stereoskopischen Bilderzeugung, mit einem Schaft, welcher eine Längsachse definiert, und mit einer Stereoskopieeinheit zur stereoskopischen Bilderzeugung. Ferner betrifft die vorliegende Erfindung ein Bildgebungssystem mit einer solchen Bildgebungsvorrichtung und ein Verfahren zum Betrieb einer solchen Bildgebungsvorrichtung.

Die Entwicklung moderner 3D-Endoskope stellt hohe Anforderungen an die präzise Ausrichtung ihrer optischen Systeme. Aus dem Stand der Technik, beispielsweise aus der US 2012/0188347 A1, sind stereoskopische Geräte einen einzelnen Bildgebungsweg aufweisend mit einem zugehörigen Sichtfeld bekannt. Dabei lehrt die US 2012/0188347 A1 verschiedene Ausgestaltungsmöglichkeiten, um unterschiedliche Bereiche des Sichtfelds zur Aufnahme von Bildern aus unterschiedlichen Perspektiven aufzunehmen.

Ferner sind aus dem Stand der Technik auch Endoskope bekannt, die zur stereoskopischen Bilderzeugung zwei räumlich voneinander getrennte optische Systeme nutzen, um einen Objektpunkt aus unterschiedlichen Blickwinkeln zu erfassen. Bei diesen Endoskopen mit zwei optischen Systemen müssen für die realistische Nachbildung der menschlichen Tiefenwahrnehmung die beiden optischen Systeme stets in einer horizontalen Lage ausgerichtet sein.

Es besteht jedoch die Problematik, dass eine Rotation des Endoskops dazu führen kann, dass eine horizontale Ausrichtung nicht mehr gegeben ist, wodurch die Augenebene des Anwenders nicht mehr mit der Ebene der beiden optischen Systeme übereinstimmt und der 3D-Eindruck verloren geht. Dies betrifft insbesondere auch Vorrichtungen, die lediglich einen einzelnen Bildgebungsweg nutzen.

Ein beispielsweise aus der US 10,365,554 B1 bekannter Lösungsansatz sieht ein Stereoendoskop mit einer Flüssigkristallschicht vor. Mittels der Flüssigkristallschicht können Blendenöffnungen für die stereoskopische Bilderfassung elektronisch festgelegt werden. Dabei wird bei einer Rotation des Endoskops die Ausrichtung bezüglich einer Referenzausrichtung des Endoskops bestimmt und darauf basierend die Position der Blendenöffnungen elektronisch angepasst. Derartige elektronische Blendenöffnungen erfordern eine komplexe Steuerungsumgebung, um die Öffnungspositionen genau zu erfassen und zu steuern. Diese Komplexität erhöht den Entwicklungsaufwand und die Produktionskosten. Faktoren wie Temperaturschwankungen, elektromagnetische Interferenzen (EMI) und materialbedingte Eigenschaften können die Leistung negativ beeinflussen.

Die Aufgabe der vorliegenden Erfindung besteht darin, eine Bildgebungsvorrichtung mit einem besonders kompakten Aufbau und vereinfachter Konstruktion zur Verfügung zu stellen, die die vorstehend genannten Probleme gemäß dem Stand der Technik zumindest teilweise löst und vorteilhaft eine drei-dimensionale Wiedergabe in unterschiedlichen Blickrichtungen der Bildgebungsvorrichtung sicherstellt.

Diese Aufgabe wird erfindungsgemäß durch eine Vorrichtung bzw. ein Verfahren mit den Merkmalen der Ansprüche 1, 14 und 15 gelöst. Vorteilhafte Ausgestaltungen und Weiterbildungen der Erfindung können den abhängigen Ansprüchen entnommen werden.

Gemäß der Erfindung wird eine Bildgebungsvorrichtung, insbesondere eine Endoskopvorrichtung, zur stereoskopischen Bilderzeugung bereitgestellt. Die Bildgebungsvorrichtung weist einen Schaft, der eine Längsachse definiert, und eine Stereoskopieeinheit zur stereoskopischen Bilderzeugung auf. Die Stereoskopieeinheit weist eine Bewegungseinheit zu einer mechanisch bewirkten Änderung eines Auswahlbereichs aus einem Sichtfeld der Stereoskopieeinheit für eine Bilderfassung auf. Die Stereoskopieeinheit ist ferner dazu eingerichtet, den Auswahlbereich zur Erfassung von Bildern zur Nutzung bei der stereoskopischen Bilderzeugung in Abhängigkeit von einem Rotationsparameter bezüglich der Längsachse festzulegen.

Durch die Möglichkeit, den Auswahlbereich mechanisch zu ändern und in Abhängigkeit von einem Rotationsparameter bezüglich der Längsachse festzulegen, kann eine Ausrichtung von Auswahlbereichen, die für die Erfassung von Bildern bei der stereoskopischen Bilderzeugung genutzt werden, zueinander horizontal gehalten werden. Somit kann eine zweckmäßige stereoskopische Bilderzeugung beispielsweise bei verschiedenen Rotationswinkels des Schafts und/oder der Stereoskopieeinheit um die Längsachse ermöglicht werden. Außerdem ist durch die mechanisch bewirkbare Änderung des Auswahlbereichs keine Notwendigkeit der Verwendung von zwei separaten optischen Systemen vorhanden. Die Verwendung von lediglich einem optischen System, insbesondere einem einzelnen Bildgebungsweg, ist für eine zweckmäßige stereoskopische Bilderzeugung, insbesondere auch bei unterschiedlichen Rotationswinkeln des Schafts, durch die erfindungsgemäße Bildgebungsvorrichtung ausreichend. Somit kann eine Bildgebungsvorrichtung mit einer vereinfachten Steuerung zur Verfügung gestellt werden. Damit kann eine besonders zuverlässige und reaktionsschnelle Anpassung der stereoskopischen Bilderzeugung an Rotationen der Bildgebungsvorrichtung erfolgen.

Die Bildgebungsvorrichtung kann als eine Endoskopvorrichtung ausgebildet sein, die als Teil eines Endoskops ausgebildet sein kann oder das gesamte Endoskop umfassen kann. Alternativ kann die Bildgebungsvorrichtung auch als Exoskopvorrichtung, insbesondere als Teil eines Exoskops ausgebildet sein oder das gesamte Exoskop umfassen. Die Bildgebungsvorrichtung ist zur stereoskopischen Bilderzeugung vorgesehen, also beispielsweise als Stereoendoskop oder Stereoexoskop ausgebildet. Die Bildgebungsvorrichtung kann dazu eingerichtet sein, Bilder eines Objektpunkts aus unterschiedlichen Perspektiven zu erfassen, um eine stereoskopische Bilderzeugung zu ermöglichen. Die Bildgebungsvorrichtung kann für medizinische oder nicht medizinische Anwendungen vorgesehen sein.

Die Stereoskopieeinheit kann zur stereoskopischen Bilderzeugung eingerichtet sein. Insbesondere kann die Stereoskopieeinheit zumindest zur Erfassung der zur stereoskopischen Bilderzeugung erforderlichen Bilder eingerichtet sein. Die Stereoskopieeinheit kann eine Sensoreinheit zur Erfassung der zur stereoskopischen Bilderzeugung erforderlichen Bilder aufweisen. Die Stereoskopieeinheit kann eine Optikeinheit zur Führung von Lichtstrahlen, und zwar insbesondere von einem zu erfassenden Objekt zu der Sensoreinheit, aufweisen. Die Stereoskopieeinheit kann eine Steuereinheit aufweisen, die dazu eingerichtet sein kann, die Erfassung von Bildern zur Nutzung bei der stereoskopischen Bilderzeugung zu steuern, beispielsweise einen Erfassungszeitpunkt und/oder Auslesezeitpunkt über die Sensoreinheit. Die Steuereinheit kann dazu eingerichtet sein, die Bewegungseinheit zu steuern. Ferner kann die Steuereinheit dazu eingerichtet sein, die mittels der Sensoreinheit erfassten Bilder für die stereoskopische Bilderzeugung zusammenzufügen. Alternativ kann auch eine zu der Steuereinheit separat ausgebildete Recheneinheit vorgesehen sein, die dazu eingerichtet sein kann, die mittels der Sensoreinheit erfassten Bilder für die stereoskopische Bilderzeugung zusammenzufügen.

Die Stereoskopieeinheit kann dazu eingerichtet sein, unterschiedliche Perspektiven für die Erfassung von Bildern eines Untersuchungsobjekts für die stereoskopische Bilderzeugung zu erzeugen, und zwar insbesondere durch die Festlegung von unterschiedlichen Auswahlbereichen. Mit anderen Worten kann die Stereoskopieeinheit zur stereoskopischen Bildererzeugung dazu eingerichtet sein, in einer Rotationsposition bezüglich der Längsachse Bilder mit unterschiedlichen und mechanisch veränderten Auswahlbereichen zu erfassen. Unter unterschiedlichen Auswahlbereichen können Auswahlbereiche mit unterschiedlichen Positionen, insbesondere bezüglich der Längsachse, verstanden werden.

Die Optikeinheit kann eine optische Blickrichtungseinheit aufweisen, die eine Blickrichtung zur Betrachtung eines Untersuchungsobjekts definiert. Die Blickrichtungseinheit kann an einem distalen Ende des Schafts angeordnet sein. Eine Anordnung der Blickrichtungseinheit am distalen Ende des Schafts kann einer Anordnung in einem distalen Endbereich des Schafts entsprechen. Mit anderen Worten muss die Blickrichtungseinheit nicht die distale Spitze des Schafts bilden. Distal vor der Blickrichtungseinheit kann ein weiteres optisches Element, beispielsweise eine Objektivoptik, ein Schutzglas oder dergleichen angeordnet sein. Die Blickrichtungseinheit kann eine oder mehrere reflektierende ebene oder gekrümmte Flächen aufweisen, beispielsweise innerhalb eines oder an einem Prisma oder zwischen mehreren Prismen.

Im Rahmen der vorliegenden Offenbarung handelt es sich bei einem proximalen Abschnitt oder einer proximalen Seite um einen Abschnitt oder eine Seite, der/die näher beim Beobachter/Anwender und weiter weg vom Sichtfeld/Patienten angeordnet ist als ein distaler Abschnitt oder eine distale Seite. Gleichermaßen handelt es sich bei einem distalen Abschnitt oder einer distalen Seite um einen Abschnitt oder eine Seite, der/die näher beim Sichtfeld/Patienten und weiter weg vom Beobachter/Anwender angeordnet ist als ein proximaler Abschnitt oder eine proximale Seite. Demgemäß kann distal auch als patientennah, dem Patienten zugewandt und/oder beobachterfern beschrieben werden. Proximal kann auch als patientenfern, dem Patienten abgewandt und/oder beobachternah beschrieben werden. Bei der Anwendung als endoskopisches Instrument ist üblicherweise das distale Ende des Schafts in den Körper eingeführt, um dort Beobachtungen vornehmen zu können. Zumindest das proximale Ende des Instruments ragt aus dem Körper heraus, weil dort die Handhabung und Steuerung durch den Bediener erfolgt.

Das Sichtfeld kann durch die Optikeinheit definiert sein, insbesondere durch die Blickrichtungseinheit. Unter einem "Sichtfeld der Stereoskopieeinheit" soll im Rahmen der Erläuterung der vorliegenden Erfindung ein durch die Optikeinheit maximaler sichtbarer Bereich bezüglich einer Blickrichtung verstanden werden. Mit anderen Worten kann das Sichtfeld einer Gesamtheit von möglichen Auswahlbereichen entsprechen. Der Auswahlbereich kann ein Teil aus dem Sichtfeld darstellen. Der Auswahlbereich kann einen Bereich, insbesondere auch eine Perspektive, bestimmen, der durch die Stereoskopieeinheit erfassbar ist, um ein Bild für die stereoskopische Bilderzeugung zu erfassen. Der von dem Auswahlbereich bestimmte Bereich kann somit einen Abbildungsbereich oder Bilderfassungsbereich definieren. Die Stereoskopieeinheit kann zur stereoskopischen Bilderzeugung dazu eingerichtet sein, Bilder mit zwei verschiedenen Auswahlbereichen, also aus zwei unterschiedlichen Perspektiven, zu erfassen, die zu einem stereoskopischen Bild zusammenfügbar sind. Der Auswahlbereich kann als eine Art Blende oder Blendenausschnitt fungieren.

Die Bewegungseinheit kann dazu eingerichtet sein, eine Position des Auswahlbereichs zu verändern. Der Auswahlbereich kann durch die Bewegungseinheit in einer Auswahlebene veränderbar sein. Die Auswahlebene kann senkrecht zur Längsachse verlaufen oder in einem Winkel größer 90° und kleiner 180° relativ zur Längsachse angeordnet sein. Das Sichtfeld kann einer Gesamtheit von möglichen Auswahlbereichen in der Auswahlebene entsprechen. Die Auswahlebene kann in einem Winkel von 90° bis 180° zu der Blickrichtung verlaufen, insbesondere in Abhängigkeit von einer Beschaffenheit oder Einstellung der Blickrichtungseinheit. Zusätzlich kann die Bewegungseinheit dazu eingerichtet sein, eine Größe und/oder Form des Auswahlbereichs verändern zu können. Der Auswahlbereich kann bezüglich seiner Größe und/oder Form alternativ auch unveränderbar sein.

Unter einer "mechanisch bewirkten Änderung" eines Auswahlbereichs soll im Rahmen der Erläuterung der vorliegenden Erfindung eine Änderung zumindest einer Position des Auswahlbereichs in der Auswahlebene und/oder relativ zur Längsachse durch eine mechanische Bewegung des Auswahlbereichs verstanden werden. Insbesondere kann die mechanisch bewirkte Änderung eine Rotationsposition des Auswahlbereichs um die Längsachse verändern, wobei die Stereoskopieeinheit die Rotationsposition in Abhängigkeit von einem Rotationsparameter bezüglich der Längsachse festlegt. Die Bewegungseinheit kann beispielsweise aktive und/oder passive Bewegungsmechanismen aufweisen. Beispielsweise kann die Bewegungseinheit einen, insbesondere hydraulischen, elektrischen, piezoelektrischen oder pneumatischen Antrieb zur mechanisch bewirkten Änderung des Auswahlbereichs aufweisen. Ein passiver Bewegungsmechanismus kann beispielsweise ein auf Federspannung, Gravitation, Elastizität oder dergleichen basierender Bewegungsmechanismus sein.

Die Steuereinheit kann dazu eingerichtet sein, den Erfassungszeitpunkt und/oder Auslesezeitpunkt über die Sensoreinheit zur Erfassung eines Bilds für die stereoskopische Bilderzeugung in Abhängigkeit von einer Position des Auswahlbereichs festzulegen. Die Steuereinheit kann dazu eingerichtet sein, die Position für den Auswahlbereich und/oder den Erfassungszeitpunkt und/oder Auslesezeitpunkt über die Sensoreinheit zur Erfassung eines Bilds für die stereoskopische Bilderzeugung in Abhängigkeit von dem Rotationsparameter bezüglich der Längsachse festzulegen. Der Rotationsparameter kann beispielsweise einen Rotationswinkel des Schafts und/oder der Stereoskopieeinheit, bzw. der Blickrichtungseinheit um die Längsachse beschreiben. Der Rotationswinkel kann beispielsweise in Bezug auf eine Ausgangslage des Schafts bestimmt werden. Die Ausgangslage kann beispielsweise durch eine Ausgangsorientierung der Stereoskopieeinheit, bzw. der Blickrichtungseinheit bestimmt sein. Beispielsweise kann eine Horizontallage einer Blickrichtung der Stereoskopieeinheit als Ausganglage gewählt werden. Grundsätzlich kann die Ausgangslage beliebig gewählt sein, zur Festlegung des Auswahlbereichs zur Erfassung von Bildern ist lediglich eine Veränderung relativ zur Ausgangslage relevant, also beispielsweise eine Veränderung des Rotationswinkels des Schafts und/oder der Stereoskopieeinheit, bzw. der Blickrichtungseinheit.

Gemäß einem bevorzugten Ausführungsbeispiel kann die Stereoskopieeinheit eine Blendeneinheit mit zumindest einer mechanisch positionierbaren Blendenöffnung aufweisen, wobei die Stereoskopieeinheit dazu eingerichtet ist, eine Position der Blendenöffnung zur Erfassung von Bildern zur Nutzung bei der stereoskopischen Bilderzeugung in Abhängigkeit von einem, insbesondere dem zuvor bereits genannten, Rotationsparameter bezüglich der Längsachse festzulegen. In einem bevorzugten Ausführungsbeispiel der Erfindung kann vorgesehen sein, dass die Blendeneinheit lediglich eine einzelne Blendenöffnung aufweist. Durch die Verwendung einer Blendeneinheit mit einer Blendenöffnung kann eine Stereoskopieeinheit mit einem besonders einfachen Aufbau bezüglich deren Steuerungsanforderungen zur Verfügung gestellt werden. Der Auswahlbereich kann durch die Blendenöffnung, insbesondere durch die Position der Blendenöffnung, definiert sein. Die Blendeneinheit kann eine Scheibe oder dergleichen aufweisen, die die Blendenöffnung aufweisen kann. Die Blendeneinheit kann bezüglich des Schafts bewegbar, insbesondere drehbar, angeordnet sein. Das Sichtfeld der Stereoskopieeinheit kann durch eine Gesamtheit der möglichen Anordnungspositionen der Blendenöffnung definiert sein. Respektive ein Auswahlbereich wird durch die Positionierung der Blendenöffnung vor dem Sichtfeld bestimmt. Ein Öffnungsbereich der Blendenöffnung ist somit kleiner als das Sichtfeld der Stereoskopieeinheit und kann den Auswahlbereich bestimmen. Durch eine Ausgestaltung der Stereoskopieeinheit mit einer Blendeneinheit, die eine mechanisch positionierbare Blendenöffnung aufweist, kann eine besonders robuste und bezüglich einer Steuerung besonders anforderungsarme Stereoskopieeinheit bereitgestellt werden.

Bei einer Ausführungsvariante der Bildgebungsvorrichtung weist die Bewegungseinheit beispielweise einen planaren Antrieb zur Translation der Blendenöffnung in einer zur Längsachse senkrechten Ebene auf. Der planare Antrieb ist vorteilhaft derart konfiguriert ist, dass er die Blendenöffnung in Abhängigkeit vom Rotationsparameter positioniert. Dabei wird die Position der Blendenöffnung in Abhängigkeit vom Rotationswinkel des Endoskops bestimmt. Der planare Antrieb ist beispielsweise ein piezoelektrischer Antrieb mit einem X-Aktor und einem Y-Aktor.

Neben einer Lösung, bei der die Stereoskopieeinheit eine Blendeneinheit aufweist, kann die Stereoskopieeinheit auch eine Reflexionseinheit mit mechanisch ausrichtbaren, insbesondere relativ zur Längsachse kippbaren, Reflexionselementen aufweisen, wobei die Stereoskopieeinheit dazu eingerichtet sein kann, eine Ausrichtung der Reflexionselemente zur Erfassung von Bildern zur Nutzung bei der stereoskopischen Bilderzeugung in Abhängigkeit von einem, insbesondere dem zuvor bereits genannten, Rotationsparameter bezüglich der Längsachse festzulegen. Die Ausgestaltung der Stereoskopieeinheit mit einer Reflexionseinheit ermöglicht einen besonders flexiblen Aufbau der Stereoskopieeinheit. Die Ausrichtung der Reflexionselemente kann den Auswahlbereich definieren. Insbesondere kann eine maximale Reflexionsfläche der Reflexionseinheit, die durch die Reflexionselemente definiert sein kann, dem Sichtfeld der Stereoskopieeinheit entsprechen. Der Auswahlbereich kann durch die Reflexionselemente definiert sein, die Lichtstrahlen eines zu erfassenden Untersuchungsobjekts auf die Sensoreinheit reflektieren. Die Reflexionseinheit kann beispielsweise als ein mikro-elektromechanisches System (MEMS) ausgebildet sein. Die Reflexionselemente können als Spiegel, insbesondere als MEMS-Spiegel, als Prismen, als Reflexionsgitter oder dergleichen ausgebildet sein.

Die Blendenöffnung der Blendeneinheit und/oder das Reflexionselement der Reflexionseinheit können als eine Art Auswahlmittel oder Auswahlelement der Bewegungseinheit zum Auswählen eines Auswahlbereichs aus dem Sichtfeld der Stereoskopieeinheit verstanden werden. Durch mechanische Änderung einer Position, insbesondere einer Umfangsposition oder Winkeleinstellung, des Auswahlmittels, bzw. Auswahlelements, relativ zur Längsachse, können unterschiedliche Auswahlbereiche ausgewählt werden. Erfindungsgemäß werden das Auswahlmittel, bzw. Auswahlelement in Abhängigkeit von einem Rotationsparameter bezüglich der Längsachse festgelegt, wie etwa einem Rotationswinkel des Schafts und/oder der Stereoskopieeinheit, bzw. der Blickrichtungseinheit um die Längsachse, wie oben beschrieben. Beispielsweise können auch die Rotationslage der Stereoskopieeinheit, die Winkelgeschwindigkeit der Blendenöffnung und/oder eine Umlaufposition der Blendenöffnung als Rotationsparameter zur Festlegung der Auswahlbereiche dienen.

Gemäß einer Weiterbildung der Erfindung kann die Stereoskopieeinheit dazu eingerichtet sein, für die stereoskopische Bilderzeugung wenigstens zwei Bilder an unterschiedlichen Auswahlbereichen, insbesondere bei unterschiedlichen Positionen der Blendenöffnung oder bei unterschiedlicher Ausrichtung der Reflexionselemente, zu erfassen. Die Stereoskopieeinheit kann insbesondere dazu eingerichtet sein, zwei Bilder mit unterschiedlichen Auswahlbereichen zu erfassen, die zwei unterschiedliche Perspektiven darstellen und für die stereoskopische Bilderzeugung zusammenfügbar sind. Dabei können die zwei Bilder an unterschiedlichen Auswahlbereichen insbesondere bei unterschiedlichen Positionen der Blendenöffnung oder bei unterschiedlicher Ausrichtung der Reflexionselemente erfasst werden. Die erfindungsgemäße Ausgestaltung der Stereoskopieeinheit ermöglicht die Erfassung von Bildern eines Untersuchungsobjekts aus unterschiedlichen Perspektiven für die stereoskopische Bilderzeugung auf besonders kompakte Weise.

Im Rahmen der Erfindung kann vorgesehen sein, dass die Stereoskopieeinheit dazu eingerichtet ist, eine Ausrichtung einer Stereobasis, die durch den Auswahlbereich, bzw. die zwei Auswahlbereiche definiert ist, bei Rotationen um die Längsachse konstant zu halten. Die Ausrichtung der Stereobasis kann somit insbesondere durch die Positionierung der Blendenöffnung oder die Ausrichtung der Reflexionselemente bei der Erfassung von Bildern für die stereoskopische Bilderzeugung definiert sein und bei Rotationen um die Längsachse konstant gehalten werden, indem der Auswahlbereich in Abhängigkeit vom Rotationsparameter bzgl. der Längsachse festgelegt wird. Somit kann erreicht werden, dass beispielsweise bei Rotationen des Schafts und/oder der Stereoskopieeinheit, bzw. der Blickrichtungseinheit um die Längsachse die Qualität der stereoskopischen Bilderzeugung erhalten werden kann.

Die Stereobasis kann durch die Positionen der Auswahlbereiche, insbesondere die Positionen der zumindest einen Blendenöffnung oder die Positionen des durch die Ausrichtung der Reflexionselemente definierten Erfassungsbereichs, definiert sein, bei denen die Bilder für die stereoskopische Bilderzeugung erfasst werden. Die Stereobasis kann insbesondere durch eine Verbindungslinie zwischen den beiden Auswahlbereichen bestimmt sein, das heißt durch eine Verbindungslinie zwischen den zwei Positionen für die zumindest eine Blendenöffnung beziehungsweise die Positionen der durch die Ausrichtung der Reflexionselemente definierten Erfassungsbereiche. Die Stereoskopieeinheit kann dazu eingerichtet sein, den Erfassungszeitpunkt und/oder Auslesezeitpunkt über die Sensoreinheit für die Erfassung der Bilder für die stereoskopische Bilderzeugung und/oder die Positionen der Auswahlbereiche derart festzulegen, dass die Stereobasis konstant ist, und zwar in einer horizontalen Lage gehalten werden kann. Darunter, dass die Stereobasis in einer horizontalen Lage gehalten wird, soll im Zusammenhang mit der Erläuterung der Erfindung verstanden werden, dass eine Parallaxe zwischen den zwei Auswahlbereichen horizontal verläuft.

Gemäß einer Weiterbildung der Erfindung kann vorgesehen sein, dass die Stereoskopieeinheit lediglich einen einzelnen Bildgebungsweg für das Empfangen der Bilder für die stereoskopische Bilderzeugung aufweist. Durch die erfindungsgemäße Ausgestaltung der Stereoskopieeinheit ist es nicht nötig, zwei separate optische Systeme, also Bildgebungswege, zu verwenden. Die Verwendung eines einzelnen Bildgebungswegs ist für eine zweckmäßige stereoskopische Bilderzeugung bei unterschiedlichen Rotationswinkeln des Schafts und/oder der Blickrichtungseinheit bezüglich der Längsachse ausreichend. Somit kann eine Bildgebungsvorrichtung zur Verfügung gestellt werden, die einen besonders einfachen und platzsparenden Aufbau aufweist. Damit kann eine Anzahl an fertigungs- und montagebedingten Fehlerquellen geringgehalten werden. Die Bildgebungsvorrichtung kann lediglich ein optisches System aufweisen, welches dazu eingerichtet sein kann, Licht von der Objektivoptik und/oder der Blickrichtungseinheit in Richtung eines proximalen Endes der Bildgebungsvorrichtung zu übertragen, und zwar vorzugsweise in Richtung der Sensoreinheit. Das optische System kann als Stablinsensystem oder als ein anderes Relaislinsensystem ausgebildet sein. Das optische System kann Teil der Optikeinheit sein.

Es kann vorgesehen sein, dass die Stereoskopieeinheit eine Lagesensorik zur Bestimmung des Rotationsparameters, insbesondere des Rotationswinkels des Schafts und/oder der Stereoskopieeinheit, bzw. der Blickrichtungseinheit, um die Längsachse aufweist. Die Stereoskopieeinheit kann dazu eingerichtet sein, den Auswahlbereich zur Erfassung von Bildern zur Nutzung bei der stereoskopischen Bilderzeugung in Abhängigkeit vom mittels der Lagesensorik erfassten Rotationsparameter bezüglich der Längsachse festzulegen. Insbesondere kann die Stereoskopieeinheit dazu eingerichtet sein, zumindest einen Erfassungszeitpunkt und/oder Auslesezeitpunkt über die Sensoreinheit für die Erfassung eines Bilds für die stereoskopische Bildererzeugung in Abhängigkeit von dem mittels der Lagesensorik erfassten Rotationsparameter festzulegen. Beispielsweise kann die Steuereinheit die Sensoreinheit derart steuern, dass sie zum Erfassungszeitpunkt und/oder Auslesezeitpunkt ein Bild des zugehörigen Auswahlbereichs erfasst. Zusätzlich oder alternativ kann die Steuereinheit dazu eingerichtet sein, die Bewegungseinheit in Abhängigkeit von dem mittels der Lagesensorik erfassten Rotationsparameter anzusteuern. Es kann auch vorgesehen sein, dass die Bewegungseinheit dazu eingerichtet ist, den Auswahlbereich fortlaufend periodisch zu ändern und die Stereoskopieeinheit lediglich einen Erfassungszeitpunkt und/oder Auslesezeitpunkt über die Sensoreinheit in Abhängigkeit von dem Rotationsparameter anpasst. Die Lagesensorik kann einen Gyrosensor, einen Beschleunigungssensor, eine inertiale Messeinheit, eine Kombination dieser oder dergleichen aufweisen. Die Lagesensorik kann zumindest datentechnisch mit der Steuereinheit verbunden sein.

Gemäß einer Weiterbildung der Erfindung kann vorgesehen sein, dass die Längsachse und die Blickrichtung der Blickrichtungseinheit einen spitzen Winkel größer Null einschließen. Der Winkel zwischen Längsachse und Blickrichtung kann als Blickwinkel bezeichnet werden. Die Bildgebungsvorrichtung kann als schrägblickendes Endoskop ausgebildet sein oder das Endoskop kann durch die Bildgebungsvorrichtung als ein schrägblickendes Endoskop ausgebildet sein. Durch die erfindungsgemäße Ausgestaltung der Stereoskopieeinheit kann eine Rotation des Schafts und/oder der Stereoskopieeinheit, bzw. der Blickrichtungseinheit um die Längsachse zur Änderung der Blickrichtung kompensiert werden, indem der Auswahlbereich, bzw. die Auswahlbereiche zur Erfassung von Bildern für die stereoskopische Bilderzeugung in Abhängigkeit der Rotation um die Längsachse angepasst werden, um eine horizontale Ausrichtung der Stereobasis zu erreichen.

In einem bevorzugten Ausführungsbeispiel der Erfindung kann vorgesehen sein, dass die Blendeneinheit ein Blendenelement mit der Blendenöffnung aufweist, das um eine Drehachse drehbar angeordnet ist. Durch eine derartige Blendeneinheit kann eine besonders robuste und bezüglich einer Steuerung besonders anforderungsarme Stereoskopieeinheit zur Verfügung gestellt werden. Das Blendenelement kann als die zuvor bereits genannte Scheibe ausgebildet sein. Gemäß einer Weiterbildung der Erfindung kann die Drehachse parallel zur Längsachse ausgerichtet sein. Insbesondere kann die Drehachse auf der Längsachse liegen. Es kann vorgesehen sein, dass die Blendenöffnung bezüglich der Drehachse exzentrisch angeordnet ist.

In einem bevorzugten Ausführungsbeispiel kann die Bewegungseinheit dazu eingerichtet sein, das Blendenelement zu einer Drehung um die Drehachse mit einer konstanten Winkelgeschwindigkeit anzutreiben. Die Bewegungseinheit kann beispielsweise einen Rotationsaktor oder dergleichen aufweisen, um das Blendenelement zu einer Drehung um die Drehachse mit der konstanten Winkelgeschwindigkeit anzutreiben. Die Bewegungseinheit kann dazu eingerichtet sein, das Blendenelement in einem Betrieb der Bildgebungsvorrichtung fortlaufend mit der konstanten Winkelgeschwindigkeit anzutreiben. Beispielsweise ist die Stereoskopieeinheit dazu eingerichtet, einen Erfassungszeitpunkt und/oder Auslesezeitpunkt über die Sensoreinheit für die Erfassung der Bilder für die stereoskopische Bilderzeugung derart festzulegen, dass sich die durch die entsprechenden Positionen der Blendenöffnung bestimmte Stereobasis in der horizontalen Lage befindet. Vorteilhaft kann vorgesehen sein, dass die Positionen der Blendenöffnung, also die Positionierung der Auswahlbereiche, an denen die Bilder für die stereoskopische Bildererzeugung erfasst werden, bezüglich der Längsachse gegenüberliegend angeordnet sind.

Es kann vorgesehen sein, dass die Reflexionseinheit um eine Achse des optischen Bildgebungswegs, insbesondere um die Längsachse, drehbar angeordnet ist. Die Bewegungseinheit kann dazu eingerichtet sein, die Reflexionseinheit, insbesondere die Reflexionselemente, um einen Winkel um die Längsachse zu drehen, der betragsmäßig einem Rotationswinkel der Drehung des Schafts und/oder der Blickrichtungseinheit um die Längsachse entspricht und insbesondere diesem Rotationswinkel entgegengesetzt ist. Alternativ kann die Reflexionseinheit relativ zu der Längsachse und/oder der Blickrichtungseinheit drehfest angeordnet sein, wobei die Reflexionselemente bezüglich der Längsachse ausrichtbar sein können. Insbesondere können die Reflexionselemente kippbar in der Reflexionseinheit gelagert sein, um bezüglich der Längsachse ausrichtbar zu sein. Eine derartige Ausgestaltung der Stereoskopieeinheit erlaubt einen besonders flexiblen Aufbau der Bildgebungsvorrichtung, die gleichzeitig eine stereoskopische Bilderzeugung bei unterschiedlichen Rotationswinkeln des Schafts und/oder der Blickrichtungseinheit um die Längsachse ermöglicht. Dabei können beispielsweise die Winkelposition der Reflexionseinheit, bzw. der Reflexionselement oder ein Kippwinkel abhängig von der Rotationslage des Schafts, bzw. der Stereoskopieeinheit zur Festlegung der Auswahlbereiche dienen.

Die Erfindung betrifft ferner ein Bildgebungssystem mit der vorstehend erläuterten erfindungsgemäßen Bildgebungsvorrichtung. Das Bildgebungssystem kann als ein Endoskopiesystem oder als ein Exoskopiesystem ausgebildet sein. Somit kann ein Bildgebungssystem bereitgestellt werden, das auch bei einer Änderung einer Blickrichtung zuverlässig eine stereoskopische Bilderzeugung ermöglicht. Durch Verwendung der erfindungsgemäßen Bildgebungsvorrichtung in dem Bildgebungssystem kann ein besonders kompaktes System zur Verfügung gestellt werden. Das Bildgebungssystem kann eine Anzeige aufweisen. Zum Zusammenfügen der mittels der Stereoskopieeinheit erfassten Bilder kann das Bildgebungssystem die Recheneinheit aufweisen. Die Recheneinheit kann zumindest einen Prozessor und ein Speicherelement sowie ein auf dem Speicherelement gespeichertes Betriebsprogramm umfassen. Das Speicherelement kann als digitales Speichermedium, beispielsweise als Speicherchip oder dergleichen, ausgebildet sein. Die Recheneinheit kann Teil der Bildgebungsvorrichtung oder separat zu der Bildgebungsvorrichtung ausgebildet sein. Die Bildgebungsvorrichtung kann über die Recheneinheit mit der Anzeige verbunden sein. Die Recheneinheit kann dazu eingerichtet sein, ein stereoskopisches Bild zur Ausgabe auf der Anzeige zu generieren. Die Anzeige kann als ein Monitor oder dergleichen ausgebildet sein. Die Recheneinheit kann mit der Steuereinheit der Bewegungseinheit datentechnisch und/oder steuerungstechnisch verbunden sein. Beispielsweise können die Recheneinheit und die Steuereinheit zumindest teilweise einstückig ausgebildet sein, insbesondere gemeinsame Bauteile aufweisen. Die Recheneinheit kann dazu eingerichtet sein, das stereoskopische Bild vor einer Ausgabe auf der Anzeige, insbesondere in Abhängigkeit von dem Rotationsparameter, zu drehen.

Ferner betrifft die Erfindung noch ein Verfahren zu einem Betrieb einer Bildgebungsvorrichtung zur stereoskopischen Bilderzeugung, insbesondere der vorstehend beschriebenen Art. In einem Verfahrensschritt, insbesondere in einem Erfassungsschritt, können Bilder mit unterschiedlichen Auswahlbereichen erfasst werden. Bei der Erfassung der Bilder kann ein erstes Bild mit einem ersten Auswahlbereich, also beispielsweise bei einer ersten Position der Blendenöffnung, erfasst werden. Mittels der durch die Bewegungseinheit erzeugten Rotation der Blendeneinheit kann der Auswahlbereich, beispielsweise eine Position der Blendenöffnung, verändert werden. Zu einem weiteren Erfassungszeitpunkt und/oder Auslesezeitpunkt über die Sensoreinheit kann bei einem zu dem ersten Auswahlbereich bezüglich einer Position verschiedenen zweiten Auswahlbereich ein zweites Bild erfasst werden. Mittels der Recheneinheit können die mittels der Sensoreinheit erfassten Bilder zur Erzeugung eines stereoskopischen Bilds zusammengefügt werden und insbesondere über die Anzeige ausgegeben werden.

In einem Verfahrensschritt, insbesondere in einem Rotationsschritt, kann die Blickrichtung der Bildgebungsvorrichtung durch eine Rotation des Schafts, insbesondere der Blickrichtungseinheit, um die Längsachse geändert werden. Gemäß dem erfindungsgemäßen Verfahren werden aus dem Sichtfeld unterschiedliche Auswahlbereiche zur Erfassung von Bildern zur Nutzung bei der stereoskopischen Bilderzeugung in Abhängigkeit von dem Rotationsparameter bezüglich der Längsachse mechanisch festgelegt. In einem bevorzugten Ausführungsbeispiel können die Auswahlbereiche in Abhängigkeit von dem Rotationsparameter so gewählt werden, dass die Stereobasis bei der Erfassung der Bilder zur Nutzung bei der stereoskopischen Bilderzeugung eine horizontale Lage aufweist. Durch ein derartiges erfindungsgemäßes Verfahren kann eine stereoskopische Bilderzeugung zuverlässig auch bei einer Änderung der Blickrichtung erfolgen. Das erfindungsgemäße Verfahren ermöglicht ferner eine besonders kompakte Ausgestaltung der Bildgebungsvorrichtung.

Die hierin offenbarten Vorrichtungen und Verfahren sollen nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf eine Vorrichtung beschriebenen Merkmale und Eigenschaften, aber auch Verfahrensweisen, sinngemäß auf Verfahren übertragbar und im Sinne der Erfindung auch als Verfahrensschritte einsetzbar sind und als solche offenbart gelten. Ebenso sind im Rahmen der vorliegenden Erfindungsbeschreibung offenbarte Verfahrensschritte als Vorrichtungsmerkmale anzusehen, die in einer Vorrichtung eingesetzt werden können. Das bedeutet, dass auch in Bezug auf Verfahren genannte, baulich also vorrichtungsgemäße Merkmale im Rahmen der Vorrichtungsansprüche berücksichtigt, beansprucht und ebenfalls zur Offenbarung gezählt werden können.

Im Folgenden wird die vorliegende Erfindung anhand der beigefügten Figuren beispielhaft beschrieben. Die Figuren, die Beschreibung und die Ansprüche enthalten zahlreiche Merkmale in Kombination. Die Fachperson wird die Merkmale zweckmäßigerweise auch einzeln betrachten und ihm Rahmen der Ansprüche sinnvoll in Kombination verwenden.

Falls von einer der nachfolgend beschriebenen Komponenten mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare der Komponente übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt jedoch kein zweites Objekt umfasst sein. Allerdings könnte anhand von Zahlenwörtern zusätzlich auch eine Anzahl und/oder eine Reihenfolge von Objekten ableitbar sein.

Es zeigen:
Fig. 1 eine schematische Darstellung eines Bildgebungssystems mit einer Anzeige, einer Recheneinheit und einer Bildgebungsvorrichtung,
Fig. 2 eine schematische Darstellung als Querschnitt eines Teils der Bildgebungsvorrichtung in einer Frontansicht,
Fig. 3 einen schematischen Ablauf eines Verfahrens zu einem Betrieb der Bildgebungsvorrichtung,
Fig. 4 eine schematische Darstellung als Querschnitt eines Teils einer Bildgebungsvorrichtung in einer ersten alternativen Ausgestaltung in einer Frontansicht und
Fig. 5 eine schematische Darstellung als Querschnitt eines Teils einer Bildgebungsvorrichtung in einer zweiten alternativen Ausgestaltung in einer Frontansicht.

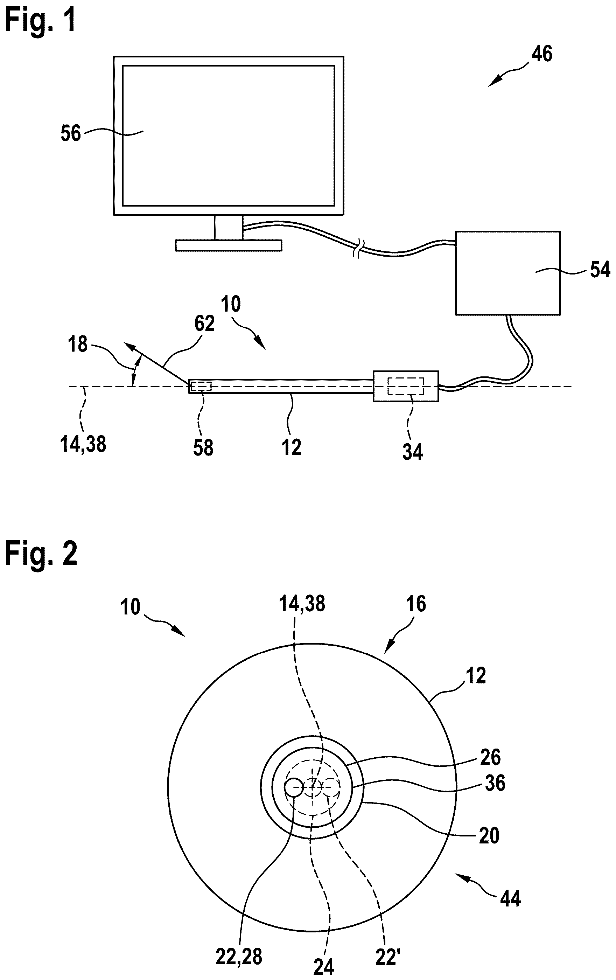

Figur 1 zeigt in einer Übersichtsdarstellung ein Bildgebungssystem 46 mit einer Bildgebungsvorrichtung 10 zur stereoskopischen Bilderzeugung, mit einer Recheneinheit 54 und mit einer Anzeige 56, welche als Monitor ausgebildet ist.

Die Bildgebungsvorrichtung 10 ist als Endoskop, insbesondere als Stereoendoskop, ausgebildet. Die Bildgebungsvorrichtung 10 weist einen Schaft 12 auf, der eine Längsachse 14 definiert.

Die Bildgebungsvorrichtung 10 weist eine Stereoskopieeinheit 16 zur stereoskopischen Bilderzeugung auf (siehe Figur 2). Die Bildgebungsvorrichtung 10, insbesondere die Stereoskopieeinheit 16, ist dazu eingerichtet, Bilder eines Objektpunkts aus unterschiedlichen Perspektiven zu erfassen, um eine stereoskopische Bilderzeugung zu ermöglichen. Die Recheneinheit 54 ist zum Zusammenfügen der mittels der Bildgebungsvorrichtung 10 erfassten Bilder aus unterschiedlichen Perspektiven für die stereoskopische Bilderzeugung eingerichtet. Die Recheneinheit 54 weist einen Prozessor (hier nicht dargestellt) und ein Speicherelement (hier nicht dargestellt) sowie ein auf dem Speicherelement gespeichertes Betriebsprogramm auf. Das Speicherelement ist als digitales Speichermedium, beispielsweise als Speicherchip oder dergleichen, ausgebildet. Die Recheneinheit 54 ist separat zu der Bildgebungsvorrichtung 10 ausgebildet. Alternativ kann die Recheneinheit 54 auch Teil der Bildgebungsvorrichtung 10 sein. Die Anzeige 56 ist datentechnisch mit der Recheneinheit 54 verbunden. Die Recheneinheit 54 ist dazu eingerichtet, ein stereoskopisches Bild zur Ausgabe auf der Anzeige 56 zu generieren.

Mit Bezug auf Figur 2 weist die Bildgebungsvorrichtung 10 eine Optikeinheit 44 zur Führung von Lichtstrahlen von einem zu erfassenden Objekt zu einer Sensoreinheit (hier nicht dargestellt) der Bildgebungsvorrichtung 10 auf. Die Optikeinheit 44 weist zumindest eine optische Blickrichtungseinheit 58 auf, die eine Blickrichtung 62 zur Betrachtung eines Untersuchungsobjekts definiert. Die Längsachse 14 und die Blickrichtung 62 schließen einen spitzen Winkel, insbesondere einen Blickwinkel 18, größer Null ein.

Die Blickrichtungseinheit 58 kann ein Prisma, mehrere Prismen oder andere Umlenkelemente zur Erzeugung des Blickwinkels von größer 0° bezüglich der Längsachse 14 aufweisen. Die Blickrichtungseinheit 58 ist an einem distalen Ende 64 des Schafts 12 angeordnet. Distal vor der Blickrichtungseinheit 58 kann eine plankonkave Linse, insbesondere zur Erzielung einer gewünschten Bildgebung, oder dergleichen angeordnet sein.

Die Stereoskopieeinheit 16 weist eine Steuereinheit (hier nicht dargestellt) auf, die dazu eingerichtet ist, die Erfassung von Bildern zur Nutzung bei der stereoskopischen Bilderzeugung zu steuern. Beispielsweise legt die Steuereinheit einen Erfassungszeitpunkt und/oder Auslesezeitpunkt für die Sensoreinheit fest. Die Stereoskopieeinheit 16 weist eine Bewegungseinheit 20 zu einer mechanisch bewirkten Änderung eines Auswahlbereichs 22, 22' aus einem Sichtfeld 24 der Stereoskopieeinheit 16 für eine Bilderfassung auf. In dem in Figur 2 gezeigten Ausführungsbeispiel ist die Bewegungseinheit 20 beispielsweise als Rotationsantrieb ausgebildet. Die Stereoskopieeinheit 16 ist dazu eingerichtet, für die stereoskopische Bilderzeugung Bilder an unterschiedlichen Auswahlbereichen 22, 22' zu erfassen, wobei die Auswahlbereiche 22, 22'insbesondere Bildern eines Untersuchungsobjekts aus unterschiedlichen Perspektiven entsprechen.

Die Steuereinheit ist dazu eingerichtet, die Bewegungseinheit 20 zu steuern. Die Steuereinheit ist Teil der Recheneinheit 54. Alternativ kann die Steuereinheit auch separat zu der Recheneinheit 54 ausgebildet sein.

In der in Figur 2 gezeigten Ausgestaltung weist die Stereoskopieeinheit 16 eine Blendeneinheit 26 mit einer mechanisch positionierbaren Blendenöffnung 28 auf. Die Blendeneinheit 26 weist ein Blendenelement 36 mit der Blendenöffnung 28 auf. Das Blendenelement 36 ist um eine Drehachse 38 drehbar angeordnet. Beispielsweise ist das Blendenelement 36 als eine kreisförmige Scheibe ausgebildet. Die Drehachse 38 liegt auf der Längsachse 14. Die Blendenöffnung 28 ist bezüglich der Drehachse 38 exzentrisch angeordnet. Die Bewegungseinheit 20 ist dazu eingerichtet, das Blendenelement 36 zu einer Drehung um die Drehachse 38 anzutreiben, vorzugsweise mit einer konstanten Winkelgeschwindigkeit. Die Bewegungseinheit 20 weist einen elektrischen Rotationsaktor zur Erzeugung der Drehung des Blendenelements 36 um die Drehachse 38 auf.

Die Stereoskopieeinheit 16 ist dazu eingerichtet, den Auswahlbereich 22, 22' zur Erfassung von Bildern zur Nutzung bei der stereoskopischen Bilderzeugung in Abhängigkeit von einem Rotationsparameter bezüglich der Längsachse 14 festzulegen. Beispielsweise bestimmt der Rotationsparameter einen Rotationswinkel des Schafts 12 und/oder der Stereoskopieeinheit 16, bzw. der Blickrichtungseinheit 58 um die Längsachse 14 und korreliert somit mit einer Blickrichtung 62 der Blickrichtungseinheit 58.

Die Stereoskopieeinheit 16 ist dazu eingerichtet, eine Ausrichtung einer Stereobasis bei der Erfassung von Bildern zur Nutzung bei der stereoskopischen Bilderzeugung bei Rotationen um die Längsachse 14 konstant zu halten. Die Stereobasis ist dabei durch zwei Auswahlbereiche 22, 22', also insbesondere zwei unterschiedliche Positionen der Blendenöffnung 28, definiert.

Die Stereoskopieeinheit 16 weist lediglich einen einzelnen Bildgebungsweg für das Empfangen der Bilder für die stereoskopische Bildererzeugung auf. Der Bildgebungsweg kann vorteilhaft auf den Auswahlbereich, insbesondere die Position der Blendenöffnung, ausgerichtet sein.

Die Stereoskopieeinheit 16 weist eine Lagesensorik 34 zur Bestimmung eines Wertes des Rotationsparameters auf. Die Lagesensorik 34 weist einen Gyrosensor, einen Beschleunigungssensor, eine inertiale Messeinheit, eine Kombination dieser oder dergleichen auf. Beispielsweise kann die Lagesensorik 34 eine Rotation des Schafts 12 um die Längsachse 14 erfassen und einen Wert eines Rotationswinkels in Bezug auf eine Ausgangslage oder Ausgangsorientierung des Schafts 14 bestimmen. Zur Festlegung des Auswahlbereichs zur Erfassung von Bildern wird eine Veränderung relative zur Ausgangslage oder relative zu einer vorhergehenden Lage erfasst. Es werden beispielsweise Werte der Rotationswinkel des Schafts und/oder der Stereoskopieeinheit, bzw. der Blickrichtungseinheit an unterschiedlichen Drehpositionen erfasst. Die Lagesensorik 34 ist zumindest datentechnisch mit der Steuereinheit verbunden.

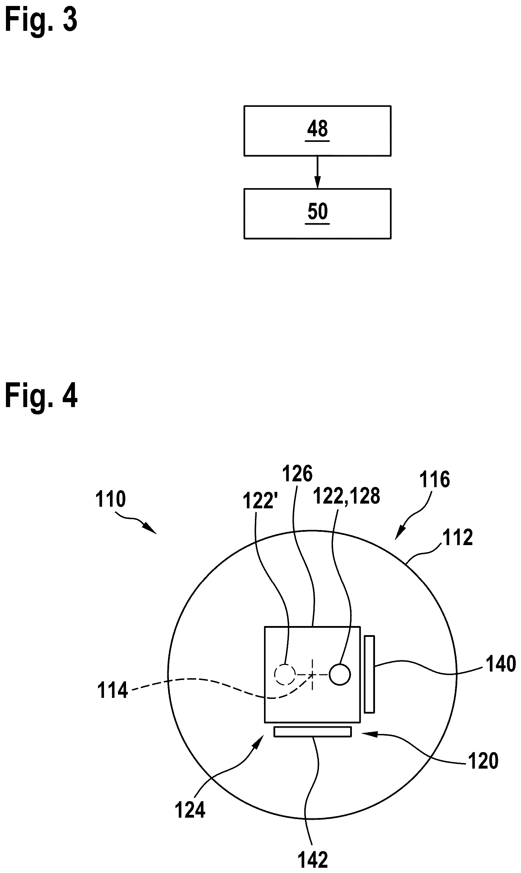

Figur 3 zeigt einen schematischen Ablauf eines Verfahrens zum Betrieb der Bildgebungsvorrichtung 10.

In einem Verfahrensschritt, insbesondere in einem Erfassungsschritt 48, werden Bilder eines Untersuchungsobjekts aus unterschiedlichen Perspektiven, also mit unterschiedlichen Auswahlbereichen 22, 22', erfasst. Bei der Erfassung der Bilder wird ein erstes Bild bei einer ersten Position der Blendenöffnung 28, also an einem ersten Auswahlbereich 22 erfasst. Durch die mittels der Bewegungseinheit 20 erzeugte Rotation der Blendeneinheit 26 wird die Position der Blendenöffnung 28 verändert, so dass die Blendenöffnung 28 eine zweite Position einnimmt und an einem zweiten Auswahlbereich 22' ein zweites Bild erfasst. Die Auswahlbereiche 22 und 22' ermöglichen eine Bilderfassung aus unterschiedlichen Perspektiven. Dabei kann die Steuereinheit einen ersten Erfassungszeitpunkt zur Aufnahme am ersten Auswahlbereich 22 und einen zweiten Erfassungszeitpunkt zur Aufnahme am zweiten Auswahlbereich 22' bestimmen. Insbesondere bei einer konstanten Umlaufgeschwindigkeit der Blendenöffnung 28 um die Drehachse 38 wird mittels der Steuereinheit ein Erfassungszeitpunkt derart festgelegt, dass der erste Auswahlbereich 22 und der zweite Auswahlbereich 22' auf einer gemeinsamen Horizontalen liegen, um die Stereobasis in horizontaler Ausrichtung zu halten. Mittels der Recheneinheit 54 werden die Bilder zu einem stereoskopischen Bild zusammengefügt und über die Anzeige 56 ausgegeben. Bei entsprechend hoher Umlaufgeschwindigkeit der Blendenöffnung 28 können ungefähr zwischen 30 bis 60 Bilder, bzw. Bilderpaare pro Sekunde erfasst werden. Für eine Live-Bildgebung kann eine Bilderfassung mit circa 120 Bilder pro Sekunde erfolgen.

In einem Verfahrensschritt, insbesondere in einem Rotationsschritt 50, wird die Blickrichtung 62 der Bildgebungsvorrichtung 10 durch eine Rotation des Schafts 12, insbesondere durch eine Rotation der Blickrichtungseinheit 58, um die Längsachse 14 geändert. Die unterschiedlichen Auswahlbereiche 22, 22' aus dem Sichtfeld 24 zur Erfassung von Bildern zur Nutzung bei der stereoskopischen Bilderzeugung werden in Abhängigkeit von dem Rotationsparameter bezüglich der Längsachse 14 festgelegt. Die Auswahlbereiche 22, 22' werden in Abhängigkeit von dem Rotationsparameter so gewählt, das heißt die Blendenöffnung 28 wird derart positioniert, dass die Stereobasis bei der Erfassung der Bilder zur Nutzung bei der stereoskopischen Bilderzeugung eine horizontale Lage aufweist. Hierfür können vorteilhaft der erste Auswahlbereich 22 und der zweite Auswahlbereich 22' relative zur Längsachse 14 einander gegenüber liegen, wobei die erste Position und die zweite Position der Blendenöffnung 28 auf einer gemeinsamen Horizontalen liegen mit der Längsachse 14 liegen können. Die Blickrichtungseinheit 58 kann somit in unterschiedliche Blickrichtungen 62 gedreht werden, die durch unterschiedliche Rotationsparameterwerte bestimmt sind, und die Stereobasis für die stereoskopische Bildgebung kann durch die erfindungsgemäße Stereoskopieeinheit horizontal gehalten werden, um einen optimalen drei-dimensionalen Bildeindruck zu erreichen.

Figur 4 zeigt eine Schnittdarstellung eines Teils einer Bildgebungsvorrichtung 110 in einer ersten alternativen Ausgestaltung. Die Bildgebungsvorrichtung 110 weist einen Schaft 112 auf, der eine Längsachse 114 definiert. Die Bildgebungsvorrichtung 110 weist eine Stereoskopieeinheit 116 zur stereoskopischen Bilderzeugung mit einer Bewegungseinheit 120 zu einer mechanisch bewirkten Änderung eines Auswahlbereichs 122, 122' aus einem Sichtfeld 124 der Stereoskopieeinheit 116 für eine Bilderfassung im Aufnahmebereich auf. Die Stereoskopieeinheit 116 ist dazu eingerichtet, den Auswahlbereich 122, 122' zur Erfassung von Bildern zur Nutzung bei der stereoskopischen Bilderzeugung in Abhängigkeit von einem Rotationsparameter bezüglich der Längsachse 114 festzulegen.

Die Stereoskopieeinheit 116 weist eine Blendeneinheit 126 mit einer Blendenöffnung 128 auf. Eine Position der Blendenöffnung 128 definiert den Auswahlbereich 122, 122'. Die Bewegungseinheit 120 weist einen planaren Antrieb zu einer Positionierung der Blendenöffnung 128 in einer Blendenebene auf. Die Blendenebene verläuft senkrecht zur Längsachse 114. Der planare Antrieb ist als ein piezoelektrischer Antrieb ausgebildet und weist einen piezoelektrischen X-Aktor 140 und einen piezoelektrischen Y-Aktor 142 auf, die in zueinander orthogonalen Achsen arbeiten. Der X-Aktor 140 ist zur Erzeugung einer Bewegung der Blendenöffnung 128 in einer X-Richtung der Blendenebene eingerichtet. Der Y-Aktor 142 ist zur Erzeugung einer Bewegung der Blendenöffnung 128 in einer Y-Richtung eingerichtet. Mittels des X-Aktors 140 und des Y-Aktors 142 kann die Blendenöffnung 128 an einer ersten Position für einen ersten Auswahlbereich 122 und an einer zweiten Position für einen zweiten Auswahlbereich 122' für unterschiedliche perspektivische Bildaufnahmen im Sichtfeld der Stereoskopieeinheit positioniert werden. Wie vorher erwähnt, werden der erste Auswahlbereich1 22 und der zweite Auswahlbereich 122', bzw. die erste Position und die zweite Position der Blendenöffnung 28 derart gewählt, dass die Auswahlbereiche für die stereoskopische Bilderzeugung auf einer gemeinsamen Horizontalen liegen.

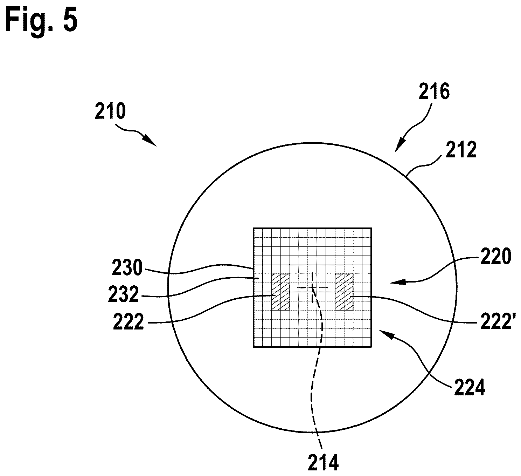

Figur 5 zeigt eine Schnittdarstellung eines Teils einer Bildgebungsvorrichtung 210 in einer zweiten alternativen Ausgestaltung. Die Bildgebungsvorrichtung 210 weist einen Schaft 212 auf, der eine Längsachse 214 definiert. Die Bildgebungsvorrichtung 210 weist eine Stereoskopieeinheit 216 zur stereoskopischen Bilderzeugung mit einer Bewegungseinheit 220 zu einer mechanisch bewirkten Änderung eines Auswahlbereichs 222, 222' aus einem Sichtfeld 224 der Stereoskopieeinheit 216 für eine Bilderfassung auf. Die Stereoskopieeinheit 216 weist eine Reflexionseinheit 230 mit einer Vielzahl an mechanisch ausrichtbaren Reflexionselementen 232 auf. Die Vielzahl der Reflexionselemente 232 können gemeinsam zumindest annähernd das Sichtfeld 224 definieren. Die Stereoskopieeinheit 216 ist dazu eingerichtet, den Auswahlbereich 222, 222' zur Erfassung von Bildern zur Nutzung bei der stereoskopischen Bilderzeugung in Abhängigkeit von einem Rotationsparameter bezüglich der Längsachse 214 festzulegen. Ein Rotationsparameterwert kann beispielsweise mit einer Lagesensorik bestimmt werden, wie vorher für die Ausgestaltungen aus Figur 2 und 4 erläutert. Die Auswahlbereiche 222, 222'sind durch die Ausrichtung der Reflexionselemente 232 definiert. Beispielsweise können die Reflexionselemente 232 durch die Bewegungseinheit 220 derart mechanisch verkippt werden, dass Licht eines Beobachtungsobjekts aus dem Auswahlbereich in den Bildgebungsweg eingespeist wird. Reflexionselemente 232 außerhalb des Auswahlbereichs leiten kein Licht in den Bildgebungsweg. Die Reflexionseinheit 230 ist um eine Achse des optischen Bildgebungswegs drehbar angeordnet. Alternativ kann die Reflexionseinheit 230 auch drehfest, beispielsweise relativ zum Schaft 212 und/oder einer Blickrichtungseinheit der Bildgebungsvorrichtung 210 angeordnet sein.

Analog zu den vorhergehenden Ausführungsbeispielen der erfindungsgemäßen Stereoskopieeinheit kann mittels der Reflexionseinheit 230 ein erster Auswahlbereich 222 und ein zweiter Auswahlbereich 222' für unterschiedliche perspektivische Bildaufnahmen im Sichtfeld der Stereoskopieeinheit eingestellt werden. Vorteilhaft werden der erste Auswahlbereich 222 und der zweite Auswahlbereich 222', bzw. die zugehörige Ausrichtung der Reflexionselemente 232 derart gewählt, dass die Auswahlbereiche für die stereoskopische Bilderzeugung auf einer gemeinsamen Horizontalen liegen.

### Bezugszeichenliste

- 10, 110, 210: Bildgebungsvorrichtung
- 12, 112, 212: Schaft
- 14, 114, 214: Längsachse
- 16, 116, 216: Stereoskopieeinheit
- 18: Blickwinkel
- 20, 120, 220: Bewegungseinheit
- 22, 122, 222: Auswahlbereich
- 24, 124, 224: Sichtfeld
- 26, 126: Blendeneinheit
- 28, 128: Blendenöffnung
- 230: Reflexionseinheit
- 232: Reflexionselemente
- 34: Lagesensorik
- 36: Blendenelement
- 38: Drehachse
- 140: X-Aktor
- 142: Y-Aktor
- 44: Optikeinheit
- 46: Bildgebungssystem
- 48: Erfassungsschritt
- 50: Rotationsschritt
- 54: Recheneinheit
- 56: Anzeige
- 58: Blickrichtungseinheit
- 62: Blickrichtung
- 64: distales Ende

## Patentansprüche

1. Bildgebungsvorrichtung (10; 110; 210), insbesondere Endoskopvorrichtung, umfassend:
- einen Schaft (12; 112; 212), der eine Längsachse (14; 114; 214) definiert, und
- eine Stereoskopieeinheit (16; 116; 216) zur stereoskopischen Bilderzeugung, **dadurch gekennzeichnet, dass** die Stereoskopieeinheit (16; 116; 216) eine Bewegungseinheit (20; 120; 220) zu einer mechanisch bewirkten Änderung eines Auswahlbereichs (22, 22'; 122, 122'; 222, 222') aus einem Sichtfeld (24; 124; 224) der Stereoskopieeinheit (16; 116; 216) für eine Bilderfassung aufweist, wobei die Stereoskopieeinheit (16; 116; 216) dazu eingerichtet ist, den Auswahlbereich (22; 122; 222) zur Erfassung von Bildern zur Nutzung bei der stereoskopischen Bilderzeugung in Abhängigkeit von einem Rotationsparameter bezüglich der Längsachse (14; 114; 214) festzulegen.

2. Bildgebungsvorrichtung (10; 110) nach dem Oberbegriff des Anspruchs 1, insbesondere nach Anspruch 1, wobei die Stereoskopieeinheit (16; 116) eine Blendeneinheit (26; 126) mit zumindest einer mechanisch positionierbaren Blendenöffnung (28; 128) aufweist, wobei die Stereoskopieeinheit (16; 116) dazu eingerichtet ist, eine Position der Blendenöffnung (28; 128) zur Erfassung von Bildern zur Nutzung bei der stereoskopischen Bilderzeugung in Abhängigkeit von einem Rotationsparameter bezüglich der Längsachse (14; 114) festzulegen.

3. Bildgebungsvorrichtung (210) nach dem Oberbegriff des Anspruchs 1, insbesondere nach Anspruch 1, wobei die Stereoskopieeinheit (216) eine Reflexionseinheit (230) mit mechanisch ausrichtbaren Reflexionselementen (232) aufweist, wobei die Stereoskopieeinheit (216) dazu eingerichtet ist, eine Ausrichtung der Reflexionselemente (232) zur Erfassung von Bildern zur Nutzung bei der stereoskopischen Bilderzeugung in Abhängigkeit von einem Rotationsparameter bezüglich der Längsachse (214) festzulegen.

4. Bildgebungsvorrichtung (10; 110; 210) nach einem der vorhergehenden Ansprüche, wobei die Stereoskopieeinheit (16; 116; 216) dazu eingerichtet ist, für die stereoskopische Bildererzeugung Bilder mit unterschiedlichen Auswahlbereichen (22, 22'; 122, 122'; 222, 222'), insbesondere bei unterschiedlichen Positionen der Blendenöffnung (28; 128) oder bei unterschiedlicher Ausrichtung der Reflexionselemente (232), zu erfassen.

5. Bildgebungsvorrichtung (10; 110; 210) nach einem der vorhergehenden Ansprüche, wobei die Stereoskopieeinheit (16; 116; 216) dazu eingerichtet ist, eine Ausrichtung einer Stereobasis, die durch den Auswahlbereich (22, 22'; 122, 122'; 222, 222'), insbesondere die Position der Blendenöffnung (28; 128) oder die Ausrichtung der Reflexionselemente (232), bei der Erfassung von Bildern zur Nutzung bei der stereoskopischen Bilderzeugung definiert ist, bei Rotationen um die Längsachse (14; 114; 214) konstant zu halten.

6. Bildgebungsvorrichtung (10; 110; 210) nach einem der vorhergehenden Ansprüche, wobei die Bewegungseinheit (120) einen planaren Antrieb zur Translation der Blendenöffnung (128) in einer zur Längsachse (114) senkrechten Ebene umfasst, wobei der planare Antrieb derart konfiguriert ist, dass er die Blendenöffnung (128) in Abhängigkeit vom Rotationsparameter positioniert.

7. Bildgebungsvorrichtung (10; 110; 210) nach dem vorhergehenden Anspruch, wobei der planare Antrieb ein piezoelektrischer Antrieb mit einem X-Aktor (140) und einem Y-Aktor (142) ist.

8. Bildgebungsvorrichtung (10; 110; 210) nach einem der vorhergehenden Ansprüche, wobei die Stereoskopieeinheit (16; 116; 216) lediglich einen einzelnen Bildgebungsweg für das Empfangen der Bilder für die stereoskopische Bilderzeugung aufweist.

9. Bildgebungsvorrichtung (10; 110; 210) nach einem der vorhergehenden Ansprüche, wobei die Stereoskopieeinheit (16; 116; 216) eine Lagesensorik (34) zur Bestimmung des Rotationsparameters aufweist.

10. Bildgebungsvorrichtung (10; 110; 210) nach einem der vorhergehenden Ansprüche, umfassend eine optische Blickrichtungseinheit (58), die eine Blickrichtung (62) zur Betrachtung eines Untersuchungsobjekts definiert, wobei die Längsachse (14; 114; 214) und die Blickrichtung (62) einen spitzen Winkel größer Null einschließen.

11. Bildgebungsvorrichtung (10) zumindest nach Anspruch 2, wobei die Blendeneinheit (26) ein Blendenelement (36) mit der Blendenöffnung (28) aufweist, das um eine Drehachse (38) drehbar angeordnet ist.

12. Bildgebungsvorrichtung (10) nach Anspruch 11, wobei die Blendenöffnung (28) bezüglich der Drehachse (38) exzentrisch angeordnet ist.

13. Bildgebungsvorrichtung (10) nach einem der Ansprüche 10 bis 12, wobei die Bewegungseinheit (20) dazu eingerichtet ist, das Blendenelement (36) zu einer Drehung um die Drehachse (38) mit einer konstanten Winkelgeschwindigkeit anzutreiben.

14. Bildgebungssystem (46), insbesondere Endoskopiesystem, mit einer Bildgebungsvorrichtung (10; 110; 210) zur stereoskopischen Bilderzeugung nach einem der vorhergehenden Ansprüche.

15. Verfahren zu einem Betrieb einer Bildgebungsvorrichtung (10; 110; 210) zur stereoskopischen Bilderzeugung, insbesondere nach einem der Ansprüche 1 bis 13, mit einem Schaft (12; 112; 212), welcher eine Längsachse (14; 114; 214) definiert, wobei aus einem Sichtfeld (24; 124; 224) unterschiedliche Auswahlbereiche (22, 22'; 122, 122'; 222, 222') zur Erfassung von Bildern zur Nutzung bei der stereoskopischen Bilderzeugung in Abhängigkeit von einem Rotationsparameter bezüglich der Längsachse (14; 114; 214) mechanisch festgelegt werden.
